# EUROPÄISCHE PATENTANMELDUNG

(11) **EP 1 106 193 A1**
(43) Veröffentlichungstag der Anmeldung: **13.06.2001**
(21) Anmeldenummer: 00121549.0
(22) Anmeldetag: 30.09.2000
(51) Int. Cl.: A61M 5/38, A61M 5/165, A61M 5/168

(54) **Infusionsgerät**

(30) Priorität: 01.12.1999 DE 29921086 U
(71) Anmelder: B. BRAUN MELSUNGEN AG, 34212 Melsungen (DE)
(72) Erfinder: Fuchs, Jürgen, 34308 Bad Emstal (DE); Harms, Volker, Dr., 34132 Kassel (DE); Dittmar, Reinhold, 34132 Kassel (DE)
(74) Vertreter: Selting, Günther, Dipl.-Ing.

(57) **Zusammenfassung**

Das Infusionsgerät weist eine Tropfkammer (10) auf, die mit einer Infusionsleitung (18) mit Schlauchklemme (20) verbunden ist. Über dem Boden (15) der Tropfkammer (10) ist eine hydrophile Filtermembran angebracht, die im befeuchteten Zustand das Eindringen von Luft in die Infusionsleitung (18) verhindert. Die Filtermembran (16) bildet einen Leerlaufstopp, mit dem bewirkt wird, dass die Infusionsleitung (18) ständig mit Flüssigkeit vollgehalten wird. Da bei Leerlaufen der Tropfkammer (10) keine Luft durch die Filtermembran (16) hindurchdringen kann, bleibt die Flüssigkeitssäule in der Infusionsleitung (18) auch bei Leerlauf der Tropfkammer (10) erhalten. Das Infusionsgerät erlaubt die Durchführung von Mehrfachinfusionen ohne den Wechsel des Infusionsgerätes. Ferner wird auch bei Lageveränderung der Tropfkammer, z.B. bei Notarzteinsätzen, ein Eindringen von Luft in die Infusionsleitung ausgeschlossen. Die Filtermembran (16) hat eine mittlere Porengröße von mehr als 10 um und besteht aus einem Material mit so hohen Kapillarkräften, dass die hydraulische Permeabilität für Wasser - bezogen auf eine Scheibe mit einem Durchmesser von 80 mm - bei einem Druck von 0,7 bar mindestens 15.000 ml/min. beträgt, wobei der Blasendruck der Filtermembran größer ist als 0,1 bar.

## Beschreibung

Die Erfindung betrifft ein Infusionsgerät mit einer Tropfkammer, von der eine zu einem Patienten führende Infusionsleitung abgeht.

Die bekannten Infusionsgeräte haben eine Tropfkammer, die am oberen Ende mit einem Einstechdorn verbunden ist. Der Einstechdorn wird durch den Verschlußstopfen eines die Infusionslösung enthaltenden Behältnisses hindurchgestochen, so dass unter Schwerkraftwirkung aus dem Behälter Infusionslösung in die Tropfkammer eintreten kann. Vom Boden der Tropfkammer geht eine Infusionsleitung ab, an der eine Dosiervorrichtung in Form einer Rollenklemme angeordnet ist, mit der die Infusionsleitung graduell abgequetscht werden kann, um die Infusionsrate zu verändern. Am patientenseitigen Ende der Infusionsleitung befindet sich ein Verbindungsstück, an welches ein Katheter oder eine andere Patientenleitung angeschlossen werden kann. Am Boden der Tropfkammer ist ein Partikelfilter angeordnet, der Feststoffpartikel in der Tropfkammer zurückhält und verhindert, dass diese Partikel in den Körper des Patienten gelangen können.

Die üblichen Infusionsgeräte können bis auf ein geringes Restvolumen in der Infusionsleitung oder sogar unter ungünstigen Umständen restlos leerlaufen. Dabei besteht die Gefahr, dass in der Tropfkammer enthaltene Luft in den Patientenkörper gelangt, was zu einer Luftembolie führen kann. Um solche Luftembolien zu vermeiden, ist es auch bei Anwendung einer Mehrfachinfusion, bei der mehrere Infusionslösungen dem Patienten nacheinander verabreicht werden, erforderlich, stets ein neues Infusionsgerät zu verwenden, wobei das alte Infusionsgerät vom Patienten abgekoppelt und durch ein neues Infusionsgerät ersetzt wird. Hierbei ist es erforderlich, nach dem Einstechen des neuen Infusionsgerätes in das entsprechende Infusionsbehältnis die Rollenklemme des alten Infusionsgerätes zu verschließen, das Verbindungsstück des alten Infusionsgerätes vom Patienten abzukoppeln, die Tropfkammer des neuen Infusionsgerätes vollaufen zu lassen und die entsprechende Infusionsleitung zu entlüften, bevor ihr Verbindungsstück an die Patientenleitung angekoppelt wird. Eine weitere Schwierigkeit besteht darin, dass die üblichen Infusionsgeräte es erfordern, dass die Tropfkammer senkrecht hängt, so dass im Tropfkammerauslaß stets eine Flüssigkeitsmenge vorhanden ist. Bei waagerechter Lage der Tropfkammer würde Luft in die Infusionsleitung gelangen und die Gefahr einer Luftembolie entstehen.

In EP 0 102 748 ist eine Vorrichtung zur Verabreichung parenteraler Lösungen beschrieben, die eine Meßkammer mit Meßskala und darunter eine Tropfkammer aufweist. Die Tropfkammer enthält eine hydrophile Filtermembran, deren Porengröße hinreichend klein ist, um zu verhindern, dass bis zu einem vorbestimmten Blasendruck Luft die Filterscheibe passiert. Der Blasendruck beträgt mindestens 0,8 bar, die nominale Porengröße beträgt etwa 0,6 µm. Hierbei handelt es sich nicht um ein' Infusionsgerät (wie z.B. in DIN 58362 Teil 1 beschrieben) zum Zuführen von Infusionslösung in das venöse Blutsystem eines Patienten. Bei der bekannten Vorrichtung zur parenteralen Ernährung ist die Filtermembran sehr dicht, so dass sich eine äußerst geringe Durchflußrate ergibt. Für höhere Durchflußraten wären sehr großflächige Filtermembrane erforderlich. Die bekannte Verabreichungsvorrichtung funktioniert nur mit geringen Durchflußraten von wenigen ml/h, wie sie in der Pädiatrie vorkommen. Durch die engporige Membran gehen keine fetthaltigen Lösungen hindurch. Solche Fettlösungen kommen jedoch in der parenteralen Ernährung vor. Für ein Infusionsgerät (wie z.B. in DIN 58362 Teil 1 beschrieben) wäre die genannte Vorrichtung ungeeignet.

Aus DE 197 48 497 A1 ist ein Infusiongerät mit Tropfkammer und Schlauchklemme bekannt, bei dem in der Tropfkammer oberhalb des Tropfkammerbodens ein hydrophiler Flüssigkeitsfilter angeordnet ist. Dieser Flüssigkeitsfilter kann eine mittlere Porengröße von weniger als 20 µm, insbesondere weniger als 15 µm, besitzen. Die mittlere Porengröße soll sogar auch von 5 bis 11 µm betragen. Die Filtermembran befindet sich nicht am Übergang der Tropfkammer zu der Infusionsleitung.

Die bekannten Infusionsgeräte mit hydrophiler Filtermembran haben den Nachteil, dass bei einer geringen Porengröße, wie sie für einen hinreichend hohen Blasendruck erforderlich ist, die Durchlässigkeit so stark eingeschränkt wird, dass bei der für Infusionen benötigten Durchflußrate eine zu große Filterfläche erforderlich wäre, so dass die gesamte Tropfkammer vergrößert werden müßte. Der Blasendruck ("bubble point") gibt denjenigen Druck an, der erforderlich ist, um die Luft durch die befeuchtete Filterscheibe zu drücken. Bei einem vorgegebenen Filtermaterial wird der Blasendruck um so höher, je geringer die Porenweite ist.

Der Erfindung liegt die Aufgabe zugrunde, ein Infusionsgerät mit hydrophiler Filtermembran zu schaffen, bei dem die Gefahr einer Luftembolie weitgehend ausgeschlossen ist und das imstande ist, bei einer relativ kleinen Fläche der Filtermembran die für Infusionen erforderliche Durchflußrate zu liefern.

Die Lösung dieser Aufgabe erfolgt erfindungsgemäß mit den im Anspruch 1 angegebenen Merkmalen.

Bei dem erfindungsgemäßen Infusionsgerät ist eine hydrophile Filtermembran vorgesehen, die das tropfkammerseitige Ende der Infusionsleitung abschließt und im befeuchteten Zustand das Eindringen von Luft in die Infusionsleitung blockiert. Dadurch wird erreicht, dass in die Infusionsleitung keine Luft eindringen kann, solange die Filtermembran mit Infusionslösung getränkt ist. Ist die Tropfkammer leergelaufen, so bildet die Filtermembran eine luftundurchlässige Sperre, durch die verhindert wird, dass Luft in die Infusionsleitung nachströmen kann. Dies bedeutet gleichzeitig, dass die in der Infusionsleitung enthaltene Flüssigkeitssäule stehenbleibt und am Absinken gehindert wird. Die Infusionsleitung bleibt also stets mit Infusionsflüssigkeit gefüllt, die bis zu der Filtermembran reicht. Hierdurch wird die Gefahr von Luftembolien erheblich verringert.

Das erfindungsgemäße Infusionsgerät ermöglicht es, Mehrfachinfusionen durchzuführen, ohne das Infusionsgerät auswechseln zu müssen. Wenn die Tropfkammer von einem ersten Infusionsbehältnis abgenommen worden ist, bleibt ein vollständiger Füllzustand der Infusionsleitung erhalten. Der Einstechdorn der Tropfkammer kann dann ohne Änderung der Einstellung der Rollenklemme in ein neues Infusionsbehältnis eingeführt werden. Nach dem Pumpen an der Tropfkammer füllt sich die Tropfkammer mit der neuen Infusionslösung und die Infusion kann fortgesetzt werden.

Die Filtermembran bewirkt einen automatischen Leerlaufstopp des Infusionsgerätes, dessen Durchfluß bei Beendigung des Zulaufs aus dem Infusionsbehältnis selbsttätig gestoppt wird, ohne dass Luft in die Infusionsleitung gelangt. Damit ist eine erhebliche Arbeitsvereinfachung für das medizinische Personal verbunden. Ferner werden die Materialkosten von Infusionen, insbesondere Mehrfachinfusionen, verringert und es wird schließlich auch eine erhebliche Verringerung des klinischen Mülls verursacht, weil weniger Geräte und zugehörige Verpackungen entsorgt werden müssen.

Ein weiterer Vorteil besteht darin, dass der Leerlaufstopp lageunabhängig funktioniert. Gelegentliche Schrägstellungen der Tropfkammer oder im Extremfall eine vertikale Lage, z.B. beim Patiententransport, führen nicht zu einer Gefahr für den Patienten.

Die Filtermembran hat eine mittlere Porengröße von mehr als 10 µm, vorzugsweise von mindestens 15 um. Diese relativ große Porengröße bewirkt eine hohe Durchlässigkeit für Flüssigkeiten. Gleichzeitig verfügt die Filtermembran über so hohe Kapillarkräfte, dass der Blasendruck der Membran größer als 0,1 bar (typische Höhendifferenz zwischen Infusionsbehälter und Patient), vorzugsweise größer als 0,3 bar, ist.

Die Erfindung ermöglicht den Einsatz einer kleinformatigen Filtermembran mit hohem Zurückhaltevermögen für die darunter befindliche Flüssigkeitssäule im Falle des Leerlaufens, bei hoher hydraulischer Durchlässigkeit im Fall des Normalbetriebes. Damit gelingt es, eine hydrophile Filtermembran in eine Tropfkammer normaler Größe zu integrieren und sowohl eine hinreichende Versorgung des Patienten mit Flüssigkeit sicherzustellen, als auch einen wirksamen Flow-Stopp im Falle des Trockenlaufens zu erreichen. Die Filtermembran befindet sich unmittelbar am Übergang von dem größeren Durchmesser der Tropfkammer in den kleineren Durchmesser der Infusionsleitung. Dies bedeutet, dass der Durchlaßquerschnitt oberhalb der Filtermembran größer ist als im darunterliegenden Leitungsabschnitt.

Vorzugsweise ist die Porosität der Filtermembran so bemessen, dass die Filtermembran Partikel mit einer Größe von mehr als 15 µm zurückhält.

Als Materialien für die Filtermembran haben sich Polysulphon und/oder Polyethersulphon besonders geeignet. Modifikationen dieser Materialien haben die erforderlichen hohen Kapillarkräfte.

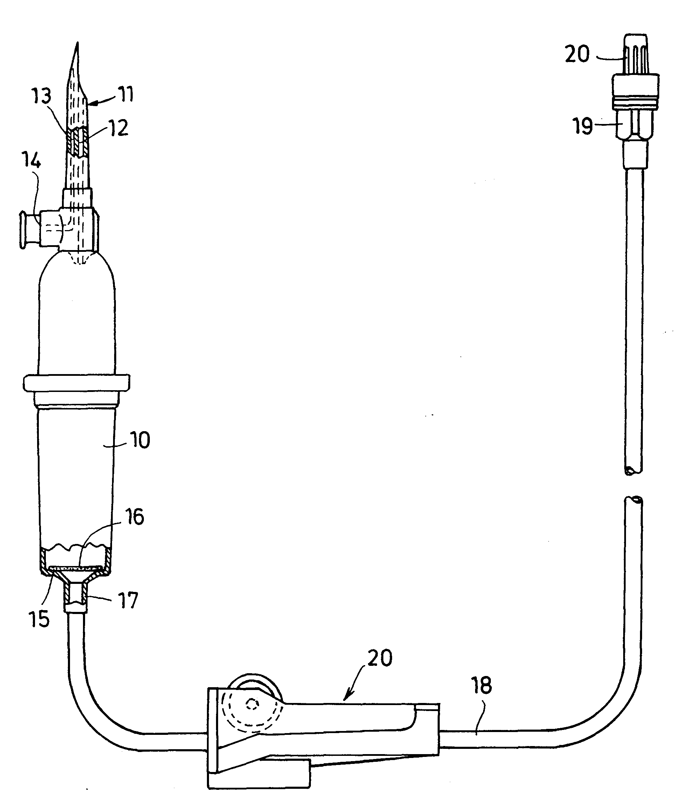

Im folgenden wird unter Bezugnahme auf die einzige Figur der Zeichnung ein Ausführungsbeispiel der Erfindung näher erläutert.

In der Zeichnung ist schematisch ein Infusionsgerät in Seitenansicht, teilweise aufgebrochen, dargestellt.

Das Infusionsgerät weist eine überlicherweise durchsichtige Tropfkammer 10 auf, die am oberen Ende mit einem Einstechdorn 11 versehen ist, welcher durch den Verschlußstopfen eines (nicht dargestellten) Infusionsbehältnisses hindurchgestochen werden kann. Durch den Einstechdorn 11 verläuft ein Kanal 12, durch den Flüssigkeit aus dem Inneren des Infusionsbehältnisses in die Tropfkammer 10 hineinläuft. Ferner ist der Einstechdorn 11 hier mit einer Belüftungsleitung 13 versehen, die mit einem Belüftungsfilter 14 verbunden ist. Durch den Belüftungsfilter 14 und die Belüftungsleitung 13 kann Luft in das Innere des Infusionsbehältnisses einströmen, um das durch die Flüssigkeitsentnahme verursachte Vakuum zu füllen. Das erfindungsgemäße Infusionsgerät kann aber auch ohne Belüftung des Infusionsbehältnisses verwendet werden, wenn das Infusionsbehältnis beispielsweise ein kollabierbarer Beutel ist.

Über dem Boden 15 der Tropfkammer 10 ist eine hydrophile Filtermembran 16 in Form einer feinporigen Scheibe oder Vliesstoffbahn angeordnet. Der Rand der Filtermembran 16 ist auf dem Boden 15 fixiert, beispielsweise durch umlaufendes Verkleben. Von dem Boden 15 geht der Tropfkammerauslaß 17 ab. Jegliches Fluid, das durch den Tropfkammerauslaß 17 fließt, muß zuvor die Filtermembran 16 passieren.

Mit dem Tropfkammerauslaß 17 ist die Infusionsleitung 18 verbunden, die aus einem flexiblen Schlauch besteht. Am patientenseitigen Ende der Infusionsleitung 18 befindet sich ein Verbindungsstück 19, das hier mit einer abnehmbaren Schutzkappe 20 verschlossen ist und beispielsweise mit einem Katheter verbunden werden kann. Auf der Infusionsleitung 18 sitzt eine übliche Schlauchklemme 20, mit der durch graduelles Abquetschen des Schlauchs die Infusionsrate verändert werden oder auch die Infusionsleitung 18 völlig verschlossen werden kann.

Nach dem Anschluß des Infusionsgerätes an ein Infusionsbehältnis wird zunächst die Infusionsleitung 18 entlüftet, indem das Ende mit dem Verbindungsstück 19 hochgehalten wird und Infusionslösung aus der Tropfkammer 10 in die Infusionsleitung läuft. Nachdem die Infusionsflüssigkeit am Verbindungsstück 19 angekommen ist und die Luft aus der Infusionsleitung herausgedrückt hat, wird das Verbindungsstück 19 mit der Patientenleitung verbunden.

Ist die Tropfkammer 10 leergelaufen, so verhindert die mit Flüssigkeit gesättigte hydrophile Filtermembran 16 den Durchgang von Luft in die Infusionsleitung 18. Dadurch wird die Infusionsleitung 18 vollständig mit Flüssigkeit vollgehalten, weil keine Luft nachströmen kann. Die in der Infusionsleitung enthaltene Flüssigkeitssäule kann durch Schwerkrafteinfluß nicht absinken.

Das Infusionsgerät ermöglicht eine Mehrfachverwendung, wobei der Einstechdorn 11 aus einem Infusionsbehältnis herausgezogen und in ein neues Infusionsbehältnis eingesteckt wird, unter Beibehaltung der an der Schlauchklemme 20 eingestellten Tropfrate. Ist der Flüssigkeitsspiegel in der Tropfkammer bis zu der Filtermembran 16 abgesunken, kommt er zum Stillstand und er steigt nach dem Einstechen in ein neues Infusionsbehältnis und dem nachfolgenden Pumpen von Flüssigkeit in die Tropfkammer wieder an.

Auch bei einer vorübergehenden Lageveränderung der Tropfkammer 19, wie sie bei Notarzteinsätzen vorkommen kann, ist ein Eindringen von Luft in die Infusionsleitung 18 ausgeschlossen.

Die Filtermembran 16 hat noch die weitere Funktion eines Partikelfilters zum Zurückhalten von Feststoffteilchen. Zweckmäßigerweise beträgt die Filtergröße 15 µm, d.h. der Filter hält alle Teilchen mit einer Partikelgröße von mehr als 15 µm zurück.

Die Filtermembran 16 hat eine mittlere Porenweite von mindestens 10 µm, vorzugsweise von 10 bis 20 µm. Es handelt sich um ein Material mit dem Hauptbestandteil Polysulphon oder Polyethersulphon. Solche Materialien sind beschrieben in den US-Patenten 5 834 107 und 5 886 059.

Das Material der Filterscheibe hat solche Kapillarkräfte, dass bei einer Porengröße von ca. 15 µm die hydraulische Permeabilität gegenüber Wasser - bezogen auf eine Scheibe mit einem Durchmesser von 80 mm - bei einem Druck von 0,7 bar, mindestens 18.000 ml/min. beträgt und der Blasendruck der Filtermembran größer als 0,1 bar, vorzugsweise größer als 0,3 bar, ist. Bei der genannten hydraulischen Permeabilität ergibt sich eine Durchflußzeit von 10 sec. für 10 ml Wasser bei einem Druck von 1 m Wassersäule, durch eine Scheibe von 12 mm Durchmesser.

Die mechanische Zugfestigkeit des Filtermaterials ist größer als 200 g und die Bruchdehnung ist größer als 15 %. Die Stärke der Filtermembran beträgt mehr als 100 um und der Blasendruck mehr als 0,3 bar.

## Patentansprüche

1. Infusionsgerät mit einer Tropfkammer (10), von der eine Infusionsleitung (18) abgeht, und einer in der Tropfkammer (10) am Übergang zu der Infusionsleitung (18) angeordneten hydrophilen Filtermembran (16), die im befeuchteten Zustand das Eindringen von Luft in die Infusionsleitung (18) blockiert,
wobei die Filtermembran (16) eine mittlere Porengröße von mehr als 10 µm hat und aus einem Material mit so hohen Kapillarkräften besteht, dass die hydraulische Permeabilität für Wasser - bezogen auf eine Scheibe mit einem Durchmesser von 80 mm - bei einem Druck von 0,7 bar mindestens 15.000 ml/min. beträgt, und dass der Blasendruck der Filtermembran größer ist als 0,1 bar.

2. Infusionsgerät nach Anspruch 1, dadurch gekennzeichnet, dass die Filtermembran (16) Partikel mit einer Größe von mehr als 15 µm zurückhält.

3. Infusionsgerät nach Anspruch 1 oder 2, dadurch gekennzeichnet, dass der Blasendruck der Filtermembran (16) größer ist als 0,3 bar.

4. Infusionsgerät nach einem der Ansprüche 1-3, dadurch gekennzeichnet, dass die Filtermembran (16) Polysulphon und/oder Polyethersulphon als Hauptbestandteil enthält.
